# FASCICULE DE BREVET EUROPEEN

(11) **EP 2 506 811 B1**
(45) Date de publication et mention de la délivrance du brevet: **15.03.2017**
(21) Numéro de dépôt: 10799065.7
(22) Date de dépôt: 29.11.2010
(51) Int. Cl.: A61F 5/01

(54) **ORTHÈSE DYNAMIQUE**
DYNAMISCHE ORTHESE
DYNAMIC ORTHOSIS

(30) Priorité: 30.11.2009 FR 0958468; 04.02.2010 FR 1050798; 17.03.2010 FR 1051885; 09.06.2010 FR 1054521
(43) Date de publication de la demande: 10.10.2012
(73) Titulaire: Teyssedre, Hervé, 1163 Etoy (CH)
(72) Inventeur: Teyssedre, Hervé, 1163 Etoy (CH)
(74) Mandataire: Cabinet Camus Lebkiri
(86) Numéro de dépôt international: PCT/FR2010/000797
(87) Numéro de publication internationale: WO 2011/067483

(56) Documents cités:
- EP-A1- 1 382 317
- DE-C- 183 418
- US-A- 4 523 394
- US-A- 5 050 620
- US-A- 5 328 444
- US-A- 5 399 152
- US-B1- 6 171 272
- US-B1- 6 793 640

## Description

La présente invention a pour objet une orthèse dynamique de pied permettant une aide à la marche, voire une rééducation fonctionnelle, notamment pour des patients atteints d'hémiplégie ou de maladies neurologiques. L'orthèse est également utilisable après l'utilisation d'un plâtre pour rééduquer le mouvement du pied à la marche.

Certaines affections musculaires ou neurologiques ont pour conséquence un pied ballant, la pointe du pied retombant naturellement vers le sol lorsque la jambe est relevée. En pratique, l'articulation de la cheville est molle, du fait d'une absence partielle ou totale de motricité musculaire. Elle ne retient pas suffisamment la pointe du pied dans une direction correspondant à une mobilité et à une motricité recherchées. Par exemple, un patient souffrant d'une telle affection doit, en marchant, lever exagérément la jambe, pour éviter de trébucher par accrochage du pied ballant sur le sol.

Les orthèses connues sont la plupart du temps des orthèses rigides ou semi-rigides, sans système dynamique indépendant généré. Le pied est bloqué dans une position perpendiculaire à la jambe. La mobilité ainsi procurée est très limitée, puisqu'elle bloque les mouvements de flexion du pied nécessaires à la marche. La démarche du patient présente une claudication visible. De plus, de telles orthèses sont malaisées à utiliser lors de la marche sur un sol accidenté, par exemple pour des promenades en montagne.

Il est connu une orthèse beaucoup plus performante à l'égard de ces problèmes de mobilité. Une telle orthèse comporte un moyen pour repousser le talon lorsque le pied est ballant, ou pour le tirer vers la jambe lorsque le pied est en appui. Une telle orthèse coopère avec le pied du patient, notamment avec l'articulation de la cheville et les articulations antérieures du pied. Il n'est donc pas nécessaire d'utiliser un organe externe d'articulation du pied, ce qui limite l'encombrement généré par l'orthèse.

Une telle orthèse comporte notamment un bracelet de jambe et une semelle de pied reliés ensemble par une structure. La structure comporte un vérin élastique déporté sur l'arrière de la jambe et du pied. Ledit vérin est extensible longitudinalement pour faire pivoter la semelle autour de l'articulation de la cheville. Une telle orthèse est décrite dans le brevet EP1382317, au nom de la Demanderesse.

Le vérin comporte typiquement une tige mobile dans un corps de vérin, ledit corps de vérin comportant une partie tubulaire. Une extrémité inférieure de la tige de vérin est par exemple reliée à la semelle de pied au niveau du talon, tandis que le corps de vérin est relié au bracelet de jambe.

Le vérin peut par exemple être un vérin pneumatique ou hydropneumatique. Il peut également s'agir d'un vérin mécanique, comportant un ressort hélicoïdal à l'intérieur du corps de vérin.

Le réglage du vérin est de préférence tel que, lorsque le pied du patient est sensiblement perpendiculaire à l'orientation de la jambe, le vérin se trouve en semi-compression. Lorsque le patient soulève sa jambe du sol, la tige du vérin se déplace vers le bas, entraînant le talon de la semelle de pied. La semelle pivote, déplaçant vers le haut la partie antérieure du pied du patient, ce qui empêche les orteils dudit pied de frotter sur le sol au cours de la marche. Lorsque le patient pose à nouveau son talon sur le sol, la tige du vérin est poussée vers le haut. La partie antérieure du pied du patient pivote vers le bas et entre en contact avec le sol.

Dans le type d'orthèses du domaine de l'invention, il est connu de relier la semelle de pied au bracelet par une ou deux sangles latérales, comme décrit dans le brevet EP1382317. Ces sangles ont pour fonction de faciliter au patient le positionnement du bracelet de jambe à la bonne hauteur. Il est connu de relier ces sangles à la semelle au niveau du talon, verticalement à l'articulation de la cheville.

Cependant, la Demanderesse a constaté qu'il était avantageux de positionner deux sangles, de part et d'autre du pied, ces sangles étant reliées à la semelle dans sa moitié antérieure, c'est-à-dire entre les orteils et un sommet de la voûte plantaire. Ces sangles sont constituées d'un matériau souple, c'est-à-dire flexible, mais non étirable.

Un tel positionnement des sangles contribue à transférer à l'avant du pied l'énergie fournie par le vérin, ce qui améliore la gestion de la position du pied par rapport au mollet au cours de la marche.

Il est également possible, en jouant sur la longueur de chacune des deux sangles, de corriger la position du pied en rotation par rapport à l'horizontale. Il est par exemple possible de corriger la supination d'un pied pour mettre la plante dudit pied en contact avec le sol.

De plus, il est avantageux de prévenir les risques d'entorses sans toutefois bloquer complètement les mouvements du pied en rotation par rapport à l'horizontale. Le fait d'équiper la semelle de deux sangles souples mais non étirables permet d'autoriser un certain angle de rotation du pied, tout en interdisant à cet angle de dépasser un seuil dangereux.

A cet effet, l'invention concerne une orthèse dynamique comportant :
- une semelle de pied,
- un bracelet de jambe,
la semelle et le bracelet étant reliés ensemble par une structure,
la structure comportant un vérin élastique déporté sur l'arrière de la jambe et du pied, une extension axiale dudit vérin permettant à la semelle de pied de pivoter autour d'une articulation d'une cheville du patient,
une partie supérieure du vérin étant reliée au bracelet par une pièce d'ancrage et une partie inférieure du vérin étant reliée au bracelet par un collier de fixation,
l'orthèse étant caractérisée en ce qu'un jeu est contenu entre le collier et ladite partie inférieure, dans un plan passant par l'axe du vérin et parallèle à un axe de marche du patient.

Dans des modes de réalisation, l'orthèse comporte une sangle reliée au bracelet sur chaque bord latéral de la jambe, chacune desdites sangles étant à la fois souple et non étirable, chacune desdites sangles étant configurée de sorte à permettre une traction sur une partie antérieure de la semelle, ladite partie étant comprise entre un aplomb des orteils et un aplomb d'un sommet de la voûte plantaire. De manière préférentielle, chacune desdites sangles est reliée à une partie antérieure de la semelle.

Selon une forme plus préférentielle de l'invention, la structure comporte en outre une sangle reliée au bracelet sur chaque bord latéral de la jambe, chacune desdites sangles étant à la fois souple et non étirable, chacune desdites sangles étant configurée de sorte à permettre une traction sur une partie postérieure de ladite semelle. Par partie postérieure, on entend la moitié de la semelle se trouvant comprise entre le talon et un aplomb d'un sommet de la voûte plantaire. De manière préférentielle, chacune desdites sangles est reliée à une partie postérieure de la semelle.

La présence de deux sangles supplémentaires à l'arrière du pied améliore le transfert de force entre le vérin et la semelle, en répartissant ladite force entre quatre points de la semelle.

De plus, le placement de quatre sangles, réparties de part et d'autre du pied et entre l'avant et l'arrière dudit pied, permet de délimiter l'amplitude de l'ensemble des mouvements anatomiques du pied par rapport à la jambe.

Comme indiqué précédemment, lorsqu'un patient portant une orthèse selon l'invention pose son talon sur le sol, la tige du vérin est poussée vers le haut. La partie antérieure du pied du patient pivote vers le bas et entre en contact avec le sol. A l'issue de cette phase de la marche, connue sous le nom de phase taligrade, l'axe de la jambe est incliné par rapport à la verticale, tandis que le pied est à l'horizontale. Le pied et la jambe forment un angle obtus. Plus le patient effectue de grands pas, plus ledit angle est important. Une grande amplitude de pas nécessite donc une grande amplitude de rotation du pied de part et d'autre de la perpendiculaire à la jambe.

L'amplitude du mouvement rotatif de la semelle par rapport à la jambe du patient dépend de la longueur de la course de la tige du vérin. Plus la course de la tige est longue, plus le pied du patient a une amplitude de mouvement importante de part et d'autre de la perpendiculaire à la jambe.

Par ailleurs, plus la course de la tige est courte, plus la pression à l'intérieur du vérin demeure importante au cours du mouvement et plus la force transmise au pied du patient est grande.

En pratique, au début du traitement d'un patient présentant une affection des muscles du pied, il est nécessaire de fournir à la semelle une force importante par l'intermédiaire du vérin. En effet, le patient n'est pas en mesure d'utiliser ses propres muscles. De plus, le manque de mobilité du patient ne lui autorise qu'une faible amplitude de pas. Le vérin est donc réglé pour une course de longueur peu importante. En pratique, le corps du vérin est fixé au bracelet de jambe dans une position assez basse.

Au fur et à mesure du déroulement du traitement, le patient peut récupérer de la puissance musculaire. Il est donc moins dépendant de la force transmise par le vérin pour déplacer son pied en rotation. De plus, la mobilité du patient s'améliorant, il est conduit à faire de plus grands pas qu'en début de traitement. Il est donc avantageux de modifier en conséquence le réglage du vérin, c'est-à-dire de fixer le corps du vérin en un point plus haut du bracelet de jambe.

Actuellement, les vérins sont reliés au bracelet de jambe par leur partie supérieure, par l'intermédiaire d'une pièce d'ancrage. Cette pièce d'ancrage est assemblée au bracelet et au vérin par des moyens de fixation de type vis. Lorsque l'on souhaite modifier le réglage de la course du vérin, il est nécessaire de démonter les vis du bracelet, de percer des trous à un nouvel emplacement choisi du bracelet et de réassembler ledit bracelet et la pièce d'ancrage. Ces manipulations doivent être réalisées par un technicien qualifié et sont difficiles à mettre en oeuvre par le médecin traitant du patient. De plus, elles imposent de perforer le bracelet de manière irréversible ; elles sont donc peu compatibles avec les réglages par tâtonnement et essais en conditions réelles qui sont couramment effectués sur ce type d'orthèses.

Il existe donc un besoin d'une orthèse permettant de modifier facilement la longueur de la course du vérin, pour accompagner la récupération de mobilité du pied d'un patient au cours du traitement.

Un objet de la présente invention est de résoudre ce problème.

Dans des modes de réalisation, une partie supérieure du vérin est reliée au bracelet par une pièce d'ancrage ; l'orthèse étant caractérisée en ce que la pièce d'ancrage comporte deux parties munies de moyens de coulissement l'une par rapport à l'autre selon un axe sensiblement parallèle à l'axe du vérin, l'une des parties étant solidaire du bracelet et l'autre partie étant solidaire de la partie supérieure du vérin, la pièce d'ancrage étant en outre munie d'un moyen de réglage réversible de la position desdites deux parties l'une par rapport à l'autre selon l'axe de coulissement.

Le moyen de réglage réversible de la position des deux parties l'une par rapport à l'autre est par exemple une vis de réglage. De cette manière, il est possible de régler progressivement la longueur de course du vérin, sans toucher à l'intégrité du bracelet de jambe. Un tel réglage ne nécessite pas d'outillage particulier et peut être effectué par le médecin traitant, par exemple au cours d'essais de l'orthèse sur un terrain accidenté.

Avantageusement, le moyen de réglage de la course du vérin est utilisé en combinaison avec les sangles telles que décrites précédemment.

Afin d'éviter des mouvements intempestifs du vérin, il est préférable de fixer au bracelet le corps dudit vérin en au moins deux points. Notamment, une partie inférieure du vérin est reliée au bracelet par un collier de fixation. Or, au cours de la marche, la distance peut varier entre le mollet et l'axe du vérin, particulièrement lorsque l'amplitude de pas du patient augmente.

Le vérin est alors soumis à des contraintes mécaniques qui peuvent entraîner une déformation, voire une rupture de la tige.

Un autre objet de l'invention est de résoudre ce problème.

En effet, l'orthèse est caractérisée en ce qu'un jeu est contenu entre le collier et la partie inférieure du vérin, dans un plan passant par l'axe du vérin et parallèle à un axe de marche du patient.

Un tel jeu permet au vérin de se déplacer légèrement par rapport à la jambe du patient, selon les mouvements de la marche. Les contraintes mécaniques subies par la tige sont donc fortement diminuées.

Selon une forme préférentielle de l'invention, afin d'autoriser ce déplacement du vérin, la fixation de la partie supérieure du vérin à la pièce d'ancrage dispose d'un degré de liberté en rotation. Il peut notamment s'agir d'une liaison pivot.

Selon une autre forme préférentielle de l'invention, la partie supérieure du vérin est bombée, en contact avec une partie bombée de la pièce d'ancrage, lesdites parties bombées étant assemblées par l'intermédiaire d'une tige traversante de diamètre inférieur à 20% d'un rayon de courbure d'une desdites parties bombées. Une certaine flexibilité ou un faible jeu de la tige autorisent un léger roulement des pièces bombées l'une contre l'autre, donc un balancement du vérin de part et d'autre de son axe principal. Ce balancement permet le déplacement de la partie inférieure du vérin selon le jeu autorisé par le collier de fixation.

Avantageusement, les moyens de balancement du vérin de part et d'autre de son axe principal sont utilisés en combinaison avec les moyens de réglage de la longueur de la course dudit vérin et/ou les sangles, tels que décrits précédemment.

Bien que de nombreux types de vérins puissent être utilisés selon l'invention, les vérins mécaniques à ressort hélicoïdal constituent une réponse optimale aux exigences dynamiques de l'orthèse.

Un tel vérin mécanique comporte un ressort hélicoïdal, contenu dans la partie tubulaire du corps de vérin. Lorsque la tige du vérin se déplace vers la partie supérieure dudit vérin, le ressort est comprimé. La vitesse de déplacement de la tige dépend de la résistance du ressort. Elle dépend également de la force exercée sur ladite tige, donc sur le talon de la semelle de pied.

Lors de la phase taligrade de la marche, le déplacement de la tige vers le haut permet à l'avant du pied d'entrer en contact avec le sol. Cependant, lorsque ce déplacement est trop rapide, les orteils s'abattent avec force sur le sol en provoquant un claquement désagréable.

Il est donc intéressant de moduler la vitesse de la course de la tige dans le corps de vérin, afin de prévenir le claquement des orteils sur le sol lors de la marche.

Dans des modes de réalisation le vérin comporte une tige et un corps de vérin contenant un ressort hélicoïdal, le vérin étant équipé d'une pièce élastique dans sa partie supérieure, de manière à offrir une résistance à la pénétration de la tige dans le corps, ladite résistance étant plus importante dans une dernière partie de la course de la tige que dans une première partie de ladite course.

La pièce élastique peut être un deuxième ressort présentant une plus grande résistance à la compression que le premier ressort. La pièce élastique peut également être un cylindre d'un matériau élastique comme le caoutchouc. Cette pièce élastique a pour fonction d'amortir la fin de la montée de la tige dans le corps de vérin. De cette manière, le déroulé du pas s'effectue de manière moins brutale et les orteils rentrent plus souplement en contact avec le sol qu'en l'absence d'une telle pièce.

Avantageusement, un vérin équipé d'une telle pièce est utilisé en combinaison avec les sangles et/ou les moyens de réglage de la course de la tige et/ou les moyens de balancement du vérin tels que décrits précédemment.

De manière classique, dans un vérin mécanique, une extrémité du ressort hélicoïdal est fixé à une partie supérieure du corps du vérin, tandis qu'une extrémité inférieure dudit ressort est fixé à la tige.

Lorsque le ressort est comprimé au maximum, les contraintes engendrent un couple de torsion qui s'exerce sur les spires du ressort. Ce couple de torsion peut conduire à la rupture du ressort par cisaillement. Or, les mouvements de l'orthèse selon l'invention conduisent à une compression fréquente et importante du ressort du vérin.

Un autre objet de l'invention est de résoudre ce problème.

Dans des modes de réalisation, le vérin comporte une tige et un corps de vérin contenant un ressort hélicoïdal, une extrémité dudit ressort étant libre en déplacement dans un plan perpendiculaire à l'axe du vérin.

Par exemple, une extrémité supérieure du ressort est libre de pivoter à l'intérieur d'une loge fixée dans la partie supérieure du corps du vérin, un plan principal de ladite loge étant perpendiculaire à l'axe du vérin. Ce degré de liberté permet de diffuser le couple de torsion lors de la compression du ressort et d'éviter la rupture dudit ressort.

Avantageusement, un tel vérin peut être équipé d'une pièce élastique amortissant la fin de la montée de la tige dans le corps de vérin, telle que décrite précédemment. De même, un tel vérin est avantageusement utilisé en combinaison avec les sangles et/ou les moyens de réglage de la course de la tige et/ou les moyens de balancement du vérin tels que décrits précédemment.

Selon une autre forme préférentielle de l'invention, lorsque la structure comporte une ou des sangles, le vérin comporte un moyen de rotation de la semelle autour d'un axe parallèle à l'axe de marche du patient. Il peut s'agir d'un moyen de type cardan, ou préférentiellement d'une rotule. Ce moyen est préférentiellement situé entre la tige du vérin et la semelle.

Selon une forme de l'invention, le terme « semelle de pied » s'entend comme une semelle disposée sous le pied du patient et susceptible d'être glissée dans une chaussure habituelle dudit patient. Selon cette forme de l'invention, l'orthèse ne nécessite pas l'utilisation de chaussures spécifiques.

Selon une autre forme de l'invention, le terme « semelle de pied » se rapporte à une semelle incorporée à une chaussure orthopédique. Préférentiellement, ladite chaussure est solidaire du reste de l'orthèse. Selon cette forme préférentielle de l'invention, une partie de l'orthèse fait donc office de chaussure pour le pied du patient.

L'invention sera mieux comprise à la lecture de la description qui suit et à l'examen des figures qui l'accompagnent. Celles-ci ne sont présentées qu'à titre indicatif et nullement limitatif de l'invention. Les figures montrent :
- Figure 1 : vue latérale d'une orthèse selon un mode de réalisation de l'invention ;
- Figure 2: vue de dessous d'une orthèse selon le mode de réalisation de l'invention représenté à la figure 1 ;
- Figure 3 : vue en perspective d'une pièce d'ancrage selon le mode de réalisation de l'invention représenté à la figure 1 ;
- Figure 4 : vue en coupe partielle d'une pièce d'ancrage assemblée à un vérin selon le mode de réalisation de l'invention représenté à la figure 1 ;
- Figure 5 : vue de dessus, en coupe, d'un collier de fixation et d'un vérin selon le mode de réalisation de l'invention représenté à la figure 1 ;
- Figure 6 : vue en coupe axiale d'un vérin selon le mode de réalisation de l'invention représenté à la figure 1.
- Figure 7 : vue latérale d'une orthèse selon un autre mode de réalisation de l'invention.

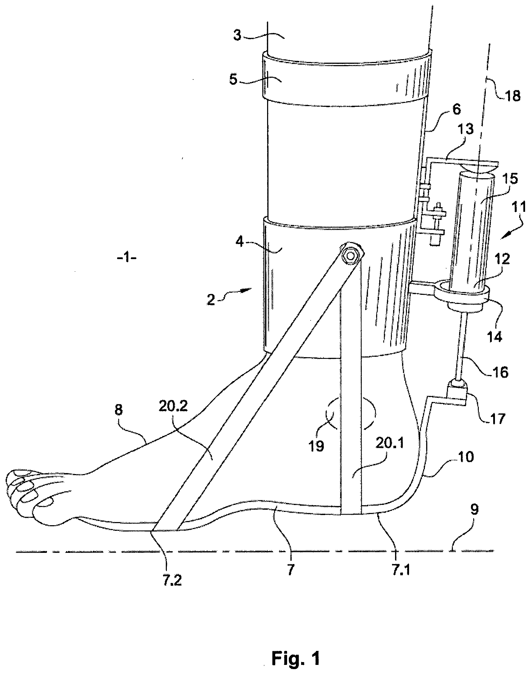

La figure 1 montre une orthèse 1 dynamique selon un mode de réalisation de l'invention, portée par un patient en station debout. L'orthèse 1 comporte un bracelet 2 destiné à être placé sur la jambe 3 d'un patient. Le bracelet 2 peut prendre plusieurs formes connues de l'état de la technique. Par exemple, le bracelet 2 peut comporter deux parties (4, 5) situées l'une au-dessus de l'autre, chaque partie (4, 5) enserrant la jambe 3, les deux parties (4, 5) étant reliées par une tige 6 sensiblement verticale, placée à l'arrière du mollet.

L'orthèse 1 comporte également une semelle 7 d'un pied 8 d'un patient. La semelle 7 peut avoir la forme d'une languette allongée sous le pied 8 et formée selon la voûte plantaire du patient. La semelle 7 peut également comporter une coque anatomique adaptée au pied 8 du patient. La semelle 7 s'étend au moins sous le talon et la voûte plantaire du pied 8. De préférence, la semelle 7 s'étend également sous une partie des os métatarsiens.

Dans l'exemple représenté à la figure 1, la semelle 7 ne s'étend pas jusqu'aux orteils du pied 8. Cependant, un mode de réalisation selon l'invention peut comporter une semelle s'étendant jusqu'aux orteils.

De manière préférentielle, la semelle 7 présente une épaisseur faible, par exemple de 2 à 5 mm. Ainsi, l'ensemble pied 8 / semelle 7 peut prendre place dans une chaussure du patient, sans que ladite chaussure ne présente d'adaptation particulière au port de l'orthèse.

On considère un axe 9 correspondant à la direction de marche du patient. On considère que la position du pied 8 est parallèle à l'axe 9. De manière préférentielle, la longueur de la semelle 7 selon l'axe 9 de la marche est égale à au moins 70% de la longueur du pied 8 selon ledit axe.

La semelle 7 comporte une talonnière 10 qui monte le long de l'arrière du talon. Le bracelet 2 est relié à la talonnière 10, donc à la semelle 7, par une structure 11. La structure 11 comporte un vérin 12 élastique, déporté sur l'arrière de la jambe 3 et du pied 8 par une pièce 13 d'ancrage. La pièce 13 est fixée d'une part à la tige 6 et d'autre part à une extrémité haute du vérin 12. En outre, une partie plus basse du vérin 12 est encerclée par un collier 14 de fixation, ledit collier étant relié au bracelet 2.

Le vérin 12 comporte un corps 15 et une tige 16, ladite tige étant apte à coulisser à l'intérieur du corps 15. Une extrémité supérieure du corps 15 est reliée à la pièce 13 d'ancrage ; une extrémité inférieure de la tige 16 est reliée à la talonnière 10 par un moyen 17 de liaison comportant plusieurs degrés de liberté en rotation. Le moyen 17 est notamment de type cardan ou rotule.

Le vérin 12 est extensible selon un axe 18, sensiblement parallèle à un axe de la jambe 3. Le coulissement de la tige 16 dans le corps 15 entraîne le pivotement de la semelle 7 autour d'une articulation 19 de la cheville du patient.

De manière préférentielle, le vérin 12 est réglé en semi-compression lorsque la jambe 3 est perpendiculaire au pied 8. Ainsi, lorsque le patient soulève son pied du sol pour faire un pas, la tige 16 coulisse vers le bas et la semelle 7 pivote de manière à écarter du sol les orteils du pied 8.

L'orthèse 1 comporte des sangles (20.1, 20.2), dont une extrémité est reliée au bracelet 2 et une autre extrémité est reliée à la semelle 7. Seules deux sangles (20.1, 20.2), disposées sur un côté visible du pied 8, sont représentées sur la figure 1. Cependant, l'orthèse 1 comporte deux autres sangles disposées de manière analogue sur l'autre côté du pied 8.

Une première sangle 20.1 est attachée à la semelle 7 au niveau d'une partie 7.1 postérieure de ladite semelle, par exemple au niveau du talon. Une seconde sangle 20.2 est attachée à la semelle 7 au niveau d'une partie 7.2 antérieure de ladite semelle, par exemple au niveau des os métatarsiens.

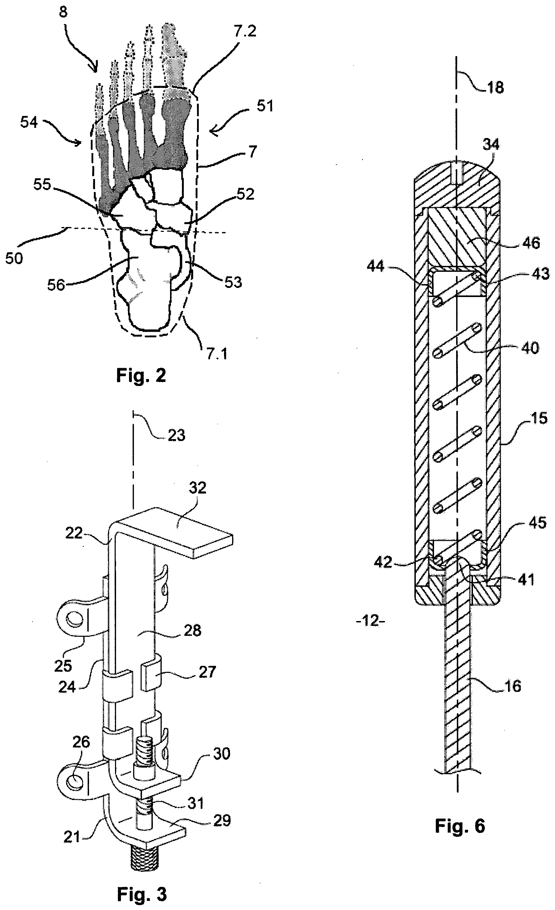

La figure 2 montre une vue schématique, de dessous, des os du pied 8. Ledit pied 8 repose sur la semelle 7 de l'orthèse 1 de la figure 1, le contour de ladite semelle étant représenté en pointillé.

Une limite 50 entre la partie 7.1 postérieure et la partie 7.2 antérieure de la semelle 7 se situe sensiblement à l'aplomb d'un sommet de la voûte plantaire du pied 8. Plus précisément, sur un bord 51 interne du pied 8, situé du côté du plus gros orteil, ladite limite 50 se trouve sensiblement entre l'os naviculaire 52 et le talus 53. Sur un bord 54 externe du pied 8, situé du côté du plus petit orteil, ladite limite 50 se trouve sensiblement entre l'os cuboïde 55 et le calcaneum 56.

L'orthèse 1 comporte quatre sangles, deux étant reliées à la partie 7.1 postérieure de la semelle 7, deux étant reliées à la partie antérieure 7.2 de ladite semelle. Cette répartition des sangles permet une transmission optimale de la force fournie par le vérin 12.

Les sangles (20.1, 20.2) sont fabriquées en matériau souple, pour pouvoir plier au cours de la marche. Cependant, les sangles (20.1, 20.2) ont notamment pour fonction de limiter les angles de torsion du pied 8 autour d'un axe parallèle à l'axe 9. En effet, de telles torsions peuvent conduire à des entorses.

Les sangles (20.1, 20.2) sont donc constituées d'un matériau non étirable, c'est-à-dire autorisant une déformation élastique quasiment nulle, notamment en traction longitudinale. Les sangles (20.1, 20.2) peuvent par exemple être en polyéthylène, en polyamide et/ou en poly-para-phénylène téréphtalamide, ce dernier étant commercialisé sous le nom de Kevlar^{®}.

Selon une variante de l'invention, les sangles en matériau de type Kevlar^{®} peuvent être remplacées par de fines bandes métalliques flexibles, montées en rotation sur le bracelet 2.

La figure 3 montre une perspective cavalière de la pièce 13 d'ancrage de l'orthèse 1. La pièce 13 comporte deux parties (21, 22) munies de moyens de coulissement l'une par rapport à l'autre selon un axe 23. Une première partie 21 comporte une bande 24 parallèle à l'axe 23, ladite bande portant des pattes 25 s'étendant perpendiculairement audit axe 23. Les pattes 25 sont percées de trous 26 et permettent l'assemblage de la pièce 13 avec le bracelet 2, par exemple au moyen de vis.

La bande 24 porte en outre des griffes 27, recourbées de manière à enserrer la seconde partie 22 de la pièce 13. La partie 22 comporte également une bande 28, parallèle à l'axe 23, de largeur sensiblement égale à celle de la bande 24. Les griffes 27 sont recourbées sur la partie 28 avec un jeu permettant aux bandes (24, 28) de coulisser l'une par rapport à l'autre selon l'axe 23.

A proximité d'une de leurs extrémités, les bandes (24, 28) sont pliées en L et se terminent respectivement par des languettes (29, 30) perpendiculaires à l'axe 23. Les languettes (29, 30) sont perforées. Une vis moletée 31, passant par ces perforations, permet de régler la position des parties (21, 22) l'une par rapport à l'autre selon l'axe 23.

Une autre extrémité de la bande 28 est également pliée en L, formant une languette 32 perpendiculaire à l'axe 23. La languette 32 est plus longue que les languettes (29, 30). Lorsque l'orthèse 1 est portée par un patient en position debout, la languette 32 est située à l'extrémité supérieure de la bande 28, tandis que les languettes (29, 30) sont situées aux extrémités inférieures des bandes (24, 28). La languette 32 est destinée à être fixée à une partie supérieure du corps 15 du vérin, comme il est visible sur la figure 1.

Ainsi, une simple manipulation de la vis 31 permet de déplacer les parties (21, 22) de la pièce 13 l'une par rapport à l'autre selon l'axe 23. Ledit déplacement permet de modifier la longueur de course du vérin 12 en fonction de l'état du patient. Ce réglage par l'intermédiaire de la vis 31 peut facilement être effectué par un médecin traitant, dans le cadre d'essais de l'orthèse.

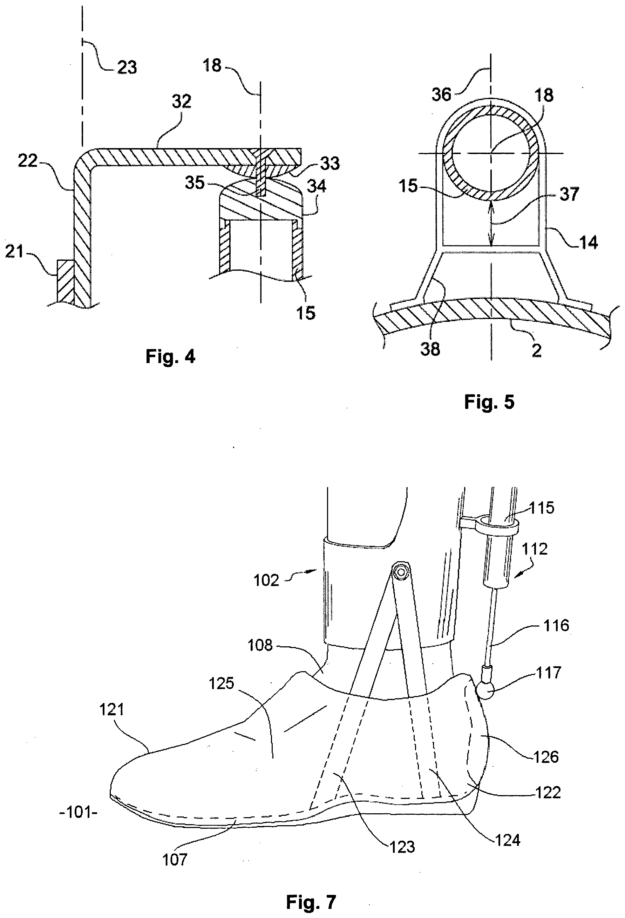

La figure 4 montre une vue en coupe partielle de la pièce 13 d'ancrage, assemblée au corps 15 du vérin 12. A proximité de son extrémité opposée à la bande 28, la languette 27 comporte une partie 33 légèrement bombée. Cette partie 33 a la forme d'une pastille, fixée sur une face de la languette 32. Ladite face de la languette 32 est celle orientée vers le bas lorsque l'orthèse 1 est portée par un patient debout.

L'extrémité supérieure du corps 15 du vérin 12 comporte un bouchon 34. Le dessus de ce bouchon 34 présente une surface légèrement bombée, avec un rayon de courbure similaire à celui de la surface 33.

La pastille 33 et le bouchon 34 sont assemblés l'un à l'autre par l'intermédiaire d'une tige 35 traversante. Les deux surfaces bombées sont donc maintenues au contact l'une de l'autre. Un diamètre de la tige 35 est inférieur à 20% d'un rayon de courbure d'une desdites surfaces bombées.

Ainsi, la tige 35 est assez fine pour avoir une certaine flexibilité, qui autorise un léger roulement des surfaces bombées l'une contre l'autre. Le corps 15 du vérin peut donc se balancer autour de sa fixation à la languette 32, décrivant par exemple un arc de cercle compris entre 2° et 5°. Ce balancement peut notamment avoir lieu dans le plan de la figure 4.

L'axe 18 principal du vérin est sensiblement parallèle à l'axe 23 de coulissement des parties (21, 22) de la pièce 13 l'une par rapport à l'autre. En raison du léger balancement du vérin 12 au cours de la marche, un angle formé par les axes (18, 23) peut subir une variation de 2° à 5° autour de sa valeur au repos. Ladite valeur au repos, soit lorsque le patient est en station debout, est notamment comprise entre 0 et 5°.

Selon un exemple de réalisation de l'invention, la tige 35 est en acier avec un diamètre de 4 mm et une longueur de 16 mm ; la pastille 33 et le bouchon 34 sont en matériau plastique, avec un rayon de courbure compris entre 24 et 28 mm.

La figure 5 représente une vue de dessus, en coupe, du collier 14 de fixation et du corps 15 du vérin. On considère que sur la figure 5, l'orthèse 1 est portée par un patient en position debout.Le collier 14, en vue de dessus, a une forme sensiblement oblongue. Dans sa partie la plus éloignée du bracelet 2, le collier 14 épouse la forme externe du corps 15 du vérin, cette forme étant celle d'un cylindre de révolution. Cependant, selon un axe 36 parallèle à l'axe 9 de la marche, une dimension interne du collier 14 est supérieure à un diamètre externe du corps 15. Ainsi, un jeu 37 permet un coulissement du corps 15 dans le collier 14 selon l'axe 36.

Le jeu 37 autorise donc un balancement du vérin 12 dans un plan passant par les axes 36 et 18, ledit plan étant perpendiculaire au plan de la figure 5.

Comme décrit précédemment, ce balancement est permis par le roulement l'une sur l'autre des surfaces bombées de la pastille 33 et du bouchon 34. Selon une autre forme de l'invention, la languette 32 est reliée au corps 15 par une liaison pivot.

Le collier 14 comporte des pattes 38 qui permettent sa fixation au bracelet 2, notamment à l'aide de vis.

La figure 6 montre une vue en coupe du vérin 12, selon un plan passant par l'axe 18 du vérin. On considère que sur la figure 5, l'orthèse 1 est portée par un patient en position debout.

Le vérin 12 comporte un corps 15 tubulaire, dans lequel peut coulisser une tige 16. Sur la figure 6, le vérin est représenté en extension maximale, c'est-à-dire que la quasi-totalité de la tige 16 est à l'extérieur du corps 15.

Le vérin 12 est un vérin mécanique. Il comporte un ressort 40 hélicoïdal à l'intérieur du corps 15.

De manière préférentielle, lorsque le vérin 12 est en extension maximale, le ressort 40 est légèrement comprimé par rapport à sa position de repos. Ainsi, le ressort 40 tend à repousser la tige 16 à l'extérieur du corps 15, quelle que soit la position de ladite tige.

Lorsque la tige 16 se déplace vers le haut dans le corps 15, l'extrémité 41 supérieure de ladite tige entraîne l'extrémité 42 inférieure du ressort 40. Les deux extrémités (42, 43) du ressort 40 se rapprochent selon l'axe 18 et les spires dudit ressort 40 se retrouvent comprimées.

Lorsque le ressort 40 est comprimé au maximum, les contraintes engendrent un couple de torsion qui peut conduire à la rupture du ressort 40 par cisaillement. Cette rupture a particulièrement lieu lorsque l'extrémité 42 inférieure du ressort est fixée à la tige 16 et que l'extrémité 43 supérieure du ressort est fixée au corps 15.

Afin de dissiper le couple de torsion lors de la compression du ressort 40, il est avantageux qu'au moins une des extrémités (42, 43) soient libres en déplacement dans un plan perpendiculaire à l'axe 18.

Dans l'exemple représenté à la figure 6, l'extrémité 43 supérieure du ressort est libre en déplacement dans une loge 44. Ladite loge a la forme d'un disque, situé dans un plan perpendiculaire à l'axe 18, ledit disque étant prolongé par une bague épousant la forme interne du corps 15 tubulaire. L'extrémité 43 est libre en déplacement contre la surface inférieure du disque de la loge 44. Ladite loge 44 est formée d'un matériau rigide, suffisamment résistant pour éviter que l'extrémité 43 ne se plante dans sa paroi.

De même, l'extrémité 42 inférieure du ressort est libre en déplacement dans une loge 45 solidaire de l'extrémité 41 de la tige 16. Lorsqu'il est comprimé, le ressort 40 peut donc tourner sur lui-même, ce qui diffuse le couple de torsion et évite des ruptures par cisaillement.

Il est également possible de laisser libre en déplacement une seule des extrémités (42, 43) du ressort, par exemple en fixant l'autre extrémité (42, 43) à sa loge (45, 44).

Lorsque la tige 16 se déplace vers le haut, comprimant le ressort 40, elle entraîne la talonnière 10 (voir figure 1), ce qui permet à l'extrémité opposée de la semelle 7 (voir figure 1) de pivoter en direction du sol.

Cependant, lorsque le déplacement de la tige 16 est trop rapide, le pivotement de la semelle 7 est brutal et provoque un claquement désagréable.

Il est donc intéressant d'amortir la course de la tige 16 dans le corps 15 lorsque l'extrémité 41 de ladite tige arrive dans une partie supérieure dudit corps 15. Ainsi, la fin du pivotement vers le sol de la semelle 7 sera plus progressive et moins bruyante.

A cet effet, une pièce 46 élastique est située dans le corps 15 tubulaire du vérin 12, entre la loge 44 et le bouchon 34 supérieur. Cette pièce 46 est par exemple constituée d'un matériau composite. Ledit matériau est sélectionné de manière à offrir une plus grande résistance à la compression que le ressort 40.

Ainsi, lorsque la tige 16 rentre dans le corps 15, le ressort 40 est comprimé de sorte que ses spires se rapprochent les unes des autres. Lorsque lesdites spires sont en contact, la progression de la tige 16 dans le corps 15 entraîne la compression de la pièce 46. Cette dernière ayant une résistance à la compression supérieure à celle du ressort 40, le déplacement de la tige 16 se trouve ralenti en fin de montée dans le corps 15.

La pièce 46 peut être remplacée par un deuxième ressort offrant une plus grande résistance à la compression que le ressort 40.

La figure 7 montre une vue latérale partielle d'une orthèse selon un autre mode de réalisation de l'invention. Ladite orthèse 101 comprend un bracelet 102 de jambe et une semelle 107 de pied. La semelle 107 est incorporée, de manière solidaire, à une chaussure 121 orthopédique, adaptée au pied 108 du patient.

L'orthèse 101 comprend en outre un vérin 112. Ledit vérin comporte un corps 115, relié au bracelet 102 par une pièce d'ancrage (non représentée) pouvant être similaire à la pièce 13 de la figure 1.

Le vérin 112 comporte en outre une tige 116, ladite tige étant apte à coulisser à l'intérieur du corps 115. Une extrémité inférieure de la tige 116 est reliée à un contrefort 122 de la chaussure 121, par un moyen 117 de liaison de type cardan ou rotule. Le vérin 112 est donc directement relié à la chaussure 121.

Préférentiellement, le moyen 117 de liaison est monté sur une pièce 126, préférentiellement métallique, incorporée dans le contrefort 122. La pièce 126 a pour fonction de transmettre les efforts entre la tige 116 du vérin et la chaussure 121.

Une orthèse 101 comprenant une chaussure 121 peut être équipée d'un ou plusieurs des dispositifs décrits ci-dessus et représentés aux figures 3, 4, 5 et 6.

De manière préférentielle, comme représenté à la figure 7, l'orthèse 101 comporte des sangles (123, 124) servant à gérer la dynamique et l'équilibre du pied du patient lors de son déplacement. Il s'agit notamment de sangles constituées d'un matériau souple mais non étirable, comme les sangles (20.1, 20.2) décrites ci-dessus en référence à la figure 1. L'orthèse 101 comporte notamment deux sangles 123, situées de part et d'autre du pied 108 et permettant une traction sur une partie antérieure de la semelle 107, ladite partie antérieure étant comprise entre un aplomb des orteils et un aplomb d'un sommet de la voûte plantaire du pied 108. Préférentiellement, une extrémité de chaque sangle 123 est reliée à la partie antérieure de la semelle 107, l'autre extrémité étant reliée au bracelet 102.

L'orthèse 101 comprend par ailleurs deux sangles 124 situées de part et d'autre du pied 108 et permettant une traction sur une partie postérieure de la semelle 107, ladite partie postérieure étant comprise entre un aplomb de la voûte plantaire et le talon du pied 108. Préférentiellement, une extrémité de chaque sangle 124 est reliée à la partie postérieure de la semelle 107, l'autre extrémité étant reliée au bracelet 102.

La chaussure 121 représentée à la figure 7 étant de profil, seules l'une des deux sangles 123 et l'une des deux sangles 124 sont visibles.

Les sangles (123, 124) peuvent passer à l'intérieur de la chaussure 121, c'est-à-dire entre le pied du patient et l'empeigne 125, ou encore à l'extérieur de la chaussure 121. Selon le mode de réalisation représenté à la figure 7, les sangles (123, 124) sont intercalées entre deux couches de matériaux constituant l'empeigne 125. Préférentiellement, une partie des sangles qui se trouve en contact avec l'empeigne 125 est solidarisée à ladite empeigne, de manière à ne pas gêner le patient par des frottements lors de la marche.

Il est préférable qu'une extrémité de chacune des sangles (123, 124) soit fixée à la semelle 107. Cependant, selon une variante de l'invention, il est possible de ne fixer ladite extrémité des sangles 123 et/ou 124 qu'à l'empeigne 125, si celle-ci est constituée d'un matériau suffisamment rigide pour transmettre efficacement un effort de traction desdites sangles (123, 124) à la semelle 107. Dans ce cas, les sangles (123, 124) sont positionnées de manière à permettre une traction, respectivement sur la partie antérieure et sur la partie postérieure de la semelle 107.

## Revendications

1. Orthèse (1, 101) dynamique comportant:
- une semelle (7, 107) de pied,
- un bracelet (2, 102) de jambe,
la semelle et le bracelet étant reliés ensemble par une structure (11),
la structure comportant un vérin (12, 112) élastique déporté sur l'arrière de la jambe et du pied, une extension axiale dudit vérin permettant à la semelle de pied de pivoter autour d'une articulation (19) d'une cheville du patient,
une partie (34) supérieure du vérin étant reliée au bracelet par une pièce (13) d'ancrage et une partie inférieure du vérin étant reliée au bracelet par un collier (14) de fixation,
l'orthèse étant **caractérisée en ce qu'**un jeu (37) est contenu entre le collier et ladite partie inférieure, dans un plan passant par l'axe (18) du vérin et parallèle à un axe (9) de marche du patient, ledit jeu permettant au vérin de se déplacer légèrement par rapport à la jambe du patient, selon les mouvements de la marche.

2. Orthèse selon la revendication 1, telle que la partie (34) supérieure du vérin est bombée, en contact avec une partie (33) bombée de la pièce (13) d'ancrage, lesdites parties bombées étant assemblées par l'intermédiaire d'une tige (35) traversante de diamètre inférieur à 20% d'un rayon de courbure d'une desdites parties bombées.

3. Orthèse selon la revendication 1 ou la revendication 2, telle que la structure (11) comporte une sangle (20.2, 123) reliée au bracelet sur chaque bord latéral de la jambe, chacune desdites sangles étant à la fois souple et non étirable, chacune desdites sangles étant configurée de sorte à permettre une traction sur une partie antérieure de la semelle, ladite partie antérieure étant comprise entre un aplomb des orteils et un aplomb d'un sommet de la voûte plantaire du pied (8).

4. Orthèse selon la revendication 3, comportant en outre une sangle (20.1, 124) reliée au bracelet sur chaque bord latéral de la jambe, chacune desdites sangles étant à la fois souple et non étirable, chacune desdites sangles étant configurée de sorte à permettre une traction sur une partie postérieure de ladite semelle, ladite partie postérieure étant comprise entre le talon et un aplomb d'un sommet de la voûte plantaire du pied (8).

5. Orthèse selon la revendication 4, telle qu'une extrémité de chaque sangle (20.1, 20.2, 123, 124) est fixée à la semelle (7, 107).

6. Orthèse selon l'une des revendications précédentes, telle que la pièce d'ancrage comporte deux parties (21, 22) munies de moyens (27) de coulissement l'une par rapport à l'autre selon un axe (23) sensiblement parallèle à un axe (18) du vérin, l'une (21) des parties étant solidaire du bracelet et l'autre (22) partie étant solidaire de la partie supérieure (34) du vérin, la pièce d'ancrage étant en outre munie d'un moyen (31) de réglage réversible de la position desdites deux parties l'une par rapport à l'autre selon l'axe (23) de coulissement.

7. Orthèse selon l'une des revendications précédentes, telle que le vérin comporte une tige (16) et un corps (15) de vérin contenant un ressort (40) hélicoïdal, le vérin étant équipé d'une pièce (46) élastique dans sa partie supérieure, de manière à offrir une résistance à la pénétration de la tige dans le corps, ladite résistance étant plus importante dans une dernière partie de la course de la tige que dans une première partie de ladite course.

8. Orthèse selon l'une des revendications précédentes, telle que le vérin comporte une tige (16) et un corps (15) de vérin contenant un ressort (40) hélicoïdal, une extrémité (43) dudit ressort étant libre en déplacement dans un plan perpendiculaire à l'axe (18) du vérin.

9. Orthèse selon l'une des revendications précédentes, telle que le vérin comporte un moyen (17, 117) de rotation de la semelle autour d'un axe parallèle à l'axe (9) de marche du patient.

10. Orthèse selon l'une des revendications précédentes, telle que la semelle (107) de pied est solidaire d'une chaussure (121).

## Patentansprüche

1. Dynamische Orthese (1, 101), umfassend:
- eine Sohle (7, 107) für einen Fuß;
- ein Band (2, 102) für das Bein;
wobei die Sohle und das Band miteinander durch eine Struktur (11) verbunden sind, wobei die Struktur einen elastischen Zylinder (12, 112) umfasst, der an der Rückseite des Beins und des Fußes versetzt ist, wobei eine axiale Erweiterung des genannten Zylinders der Sohle für den Fuß ein Schwenken rund um eine Artikulation (19) eines Fußgelenks des Patienten erlaubt,
wobei ein oberer Teil (34) des Zylinders durch ein Verankerungsteil (13) mit dem Zylinder verbunden ist und ein unterer Teil des Zylinders durch eine Befestigungsschelle (14) mit dem Band verbunden ist,
wobei die Orthese **dadurch gekennzeichnet ist, dass** ein Spiel (37) zwischen der Schelle und dem genannten unteren Teil in einer Ebene enthalten ist, die durch die Achse (18) des Zylinders und parallel zu einer Laufachse (9) des Patienten hindurchtritt, wobei es das genannte Spiel dem Zylinder ermöglicht, sich im Verhältnis zum Bein des Patienten gemäß den Bewegungen des Gehens leicht zu verschieben.

2. Orthese gemäß Anspruch 1 derart, dass der obere Teil (34) des Zylinders im Kontakt mit einem gewölbten Teil (33) des Verankerungsteils (13) gewölbt ist, wobei die genannten gewölbten Teile mittels eines durchlaufenden Stiftes (35) mit einem Durchmesser von weniger als 20 % eines Krümmungsradius eines der genannten gewölbten Teile zusammengesetzt sind.

3. Orthese gemäß Anspruch 1 oder Anspruch 2 derart, dass die Struktur (11) einen Gurt (20.2, 123) umfasst, der mit dem Band an jedem lateralen Rand des Beins verbunden ist, wobei jedes der genannten Bänder gleichzeitig weich und nicht dehnbar ist, wobei jedes der genannten Bänder derart konfiguriert ist, dass ein Zug auf einem vorderen Teil der Sohle zugelassen wird, wobei der genannte vordere Teil zwischen einem Lot der Zehen und einem Lot einer Spitze des Fußgewölbes (8) inbegriffen ist.

4. Orthese gemäß Anspruch 3, umfassend darüber hinaus einen Gurt (20.1, 124), der am Band an jedem lateralen Rand des Bens verbunden ist, wobei jeder der genannten Gurte gleichzeitig weich und nicht dehnbar ist, wobei jeder der genannten Gurte derart konfiguriert ist, dass ein Zug auf einem hinteren Teil der genannten Sohle zugelassen wird, wobei der genannte hintere Teil zwischen der Ferse und einem Lot einer Spitze des Fußgewölbes (8) inbegriffen ist.

5. Orthese gemäß Anspruch 4 derart, dass ein Ende jedes Gurtes (20.1, 20.1, 123, 124) an der Sohle (7, 107) befestigt ist.

6. Orthese gemäß einem der voranstehenden Ansprüche, derart, dass das Verankerungsteil zwei Teile (21, 22) umfasst, die mit Mitteln (27) zum Gleiten im Verhältnis zueinander gemäß einer Achse (23) deutlich parallel zu einer Achse (18) des Zylinders versehen sind, wobei einer (21) der Teile mit dem Band fest verbunden ist und der andere (22) mit dem oberen Teil (34) des Zylinders fest verbunden ist, wobei das Verankerungsteil darüber hinaus mit einem reversiblen Einstellmittel (31) der Position der genannten zwei Teile im Verhältnis zueinander gemäß der Achse (23) zum Gleiten versehen ist.

7. Orthese gemäß einem der voranstehenden Ansprüche derart, dass der Zylinder einen Stift (16) und ein Zylinderkörper (15), der eine spiralförmige Feder (40) enthält, umfasst, wobei der Zylinder in seinem oberen Teil mit einem elastischen Teil (46) derart versehen ist, dass ein Widerstand gegen das Eindringen des Stiftes in den Körper geboten wird, wobei der genannte Widerstand in einem letzten Teil des Hubs des Stiftes größer ist als in einem ersten Teil des genannten Hubs.

8. Orthese gemäß einem der voranstehenden Ansprüche derart, dass der Zylinder einen Stift (16) und einen Zylinderkörper (15) umfasst, der eine spiralförmige Feder (40) umfasst, wobei ein Ende (43) der genannten Feder in der Verschiebung in einer lotrechten Ebene der Achse (18) des Zylinders frei ist.

9. Orthese gemäß einem der voranstehenden Ansprüche derart, dass der Zylinder ein Rotationsmittel (17, 117) der Sohle um eine Achse umfasst, die parallel zur Laufachse (9) des Patienten ist.

10. Orthese gemäß einem der voranstehenden Ansprüche derart, dass die Sohle (107) des Fußes fest mit einem Schuh (121) verbunden ist.

## Claims

1. A dynamic orthosis (1, 101) including:
- a foot sole (7, 107),
- a leg bracelet (2, 102),
the sole and the bracelet being connected to each other by a structure (11), the structure including an elastic actuator (12, 112) rearwardly offset from the leg and foot, an axial extension of said actuator allowing the foot sole to pivot about a joint (19) of a patient's ankle,
an upper part (34) of the actuator being connected to the bracelet by an anchoring piece (13) and a lower part of the actuator being connected to the bracelet by an attaching collar (14),
the orthosis being **characterized in that** a clearance (37) is contained between the collar and said lower part, in a plane passing through the axis (18) of the actuator and parallel to a walking axis (9) of the patient, said clearance allowing the actuator to slightly move with respect to the patient's leg, according to the walking motions.

2. The orthosis according to claim 1, wherein the upper part (34) of the actuator is domed, in contact with a domed part (33) of the anchoring piece (13), said domed parts being assembled via a through rod (35) with a diameter smaller than 20 % of a radius of curvature of one of said domed parts.

3. The orthosis according to claim 1 or claim 2, wherein the structure (11) includes a strap (20.2, 123) connected to the bracelet on each said edge of the leg, each of said straps being both flexible and non stretchable, each of said straps being configured so as to allow a traction on an anterior part of the sole, said anterior part being included between a plumb of the toes and a plumb of an apex of the arch of the foot (8).

4. The orthosis according to claim 3, further including a strap (20.1, 124) connected to the bracelet on each side edge of the leg, each of said straps being both flexible and non stretchable, each of said straps being configured so as to allow a traction on a posterior part of said sole, said posterior part being included between the heel and a plumb of an apex of the arch of the foot (8).

5. The orthosis according to claim 4, wherein an end of each strap (20.1, 20.2, 123, 124) is attached to the sole (7, 107).

6. The orthosis according to one of the previous claims, wherein the anchoring piece includes two parts (21, 22) provided with means (27) for sliding with respect to each other along an axis (23) substantially parallel to an axis (18) of the actuator, one (21) of the parts being integral with the bracelet and the other part (22) being integral with the upper part (34) of the actuator, the anchoring piece being further provided with a means (31) for reversibly adjusting the position of both said parts with respect to each other along the sliding axis (23).

7. The orthosis according to one of the previous claims, wherein the actuator includes an actuator rod (16) and an actuator body (15) containing a coil spring (40), the actuator being fitted with an elastic piece (46) in its upper part, so as to provide resistance to penetration of the rod into the body, said resistance being greater in a last part of the rod stroke than in a first part of said stroke.

8. The orthosis according to one of the previous claims, wherein the actuator includes an actuator rod (16) and an actuator body (15) containing a coil spring (40), one end (43) of said spring being freely moving in a plane perpendicular to the axis (18) of the actuator.

9. The orthosis according to one of the previous claims, wherein the actuator includes a means (17, 117) for rotating the sole about an axis parallel to the walking axis (9) of the patient.

10. The orthosis according to one of the previous claims, wherein the foot sole (107) is integral with a shoe (121).
